# EUROPEAN PATENT APPLICATION

(11) **EP 4 635 534 A1**
(43) Date of publication of application: **22.10.2025**
(21) Application number: 23848266.5
(22) Date of filing: 13.12.2023
(51) Int. Cl.: A61M 5/20, A61M 5/28, A61M 5/315, A61M 5/31, A61M 5/32

(54) **PORTABLE ADRENALINE AUTO INJECTOR AND METHOD OF OPERATION OF THE AUTO INJECTOR DEVICE**

(30) Priority: 14.12.2022 PT 2022118396
(71) Applicant: Universidade de Aveiro, 3810-193 Aveiro (PT); Unidade Local de Saúde de São João E.P.E., 4200-319 Porto (PT)
(72) Inventor: ANTÓNIO OLIVEIRA FERNANDES, Fábio, 4520-238 SANTA MARIA DA FEIRA (PT); JORGE HENRIQUES NORONHA, Eduardo, 3830-472 GAFANHA DA ENCARNAÇÃO (PT); ALEXANDRA MIGUEL SERRANO, Clara, 8500-520 PORTIMÃO (PT); DANIEL VALENTE FONSECA, Ivo, 3810-554 AVEIRO (PT); SOLEDADE RIBEIRO COIMBRA PEIXOTO, Alice, 4050-042 PORTO (PT)
(74) Representative: Monteiro Alves, Inês
(86) International application number: PCT/PT2023/050044
(87) International publication number: WO 2024/128930

(57) **Abstract**

The adrenaline auto injector of the present invention is a portable analog apparatus which contains an adrenaline dose for intramuscular injection, which allows the user or third parties to administer the solution in situations of imminent anaphylactic reaction.

Said device presents a protective semi-rigid membrane of the button which triggers the administration of the adrenaline dose. This semi-rigid membrane, positioned at the opposite end of the orifice for expelling the needle, has the double function of protecting the button against inadvertent use and allowing, simultaneously, access to the button at the moment of application of adrenaline, without the need for interacting with movable parts of the device for unblocking the needle protection mechanism, being necessary only two steps to administer the adrenaline dose.

## Description

### TECHNICAL DOMAIN OF THE INVENTION

The present invention is related to the technical field of medical use devices, concretely an adrenaline auto injector device.

### Background of the Invention and Problems of the Prior Art

Anaphylaxis is a generalized or systemic severe allergic reaction, which is potentially fatal, characterized by the rapid beginning of breathing and/or circulatory difficulties, generally associated with skin reactions (urticaria) and/or of the mucous (edema). Patients with severe allergies are counseled to carry two adrenaline auto injectors, ready for administration if necessary. Morphologically, most of the devices present an elongated configuration, similar to the appearance of a pen, and foresee, for the administration of the treatment, a collision movement of the device against the thigh, which triggers the release of the needle and injects adrenaline in the muscle. All the devices have one or two safety pins which the user must remove for the device to work. Not removing the safety pin is one of the most common errors. This is due to the fact that the user, particularly in high stress situations, cannot understand that these small protection pins must be removed before injecting, causing even more stress or even inability to administer the dose.

Patent US8870827B2 describes an automatic auto injector with a needle cover mechanism to avoid a user contacting the needle of the auto injector before use. The needle cover mechanism is locked prior to activating the auto injector. After use of the injector, the needle cover mechanism remains held in a locked position to prevent the user from accessing the needle. The patent describes an outer body which presents at one of the ends an opening to receive a release pin. When in place, the release pin prevents the inadvertent use or the activation of the auto injector. The container which houses the pin presents two side slits so as to receive the tabs of the release pin. The tabs prevent rotation of the release pin such that the user easily recognizes that the release pin must be pulled instead of rotated to allow the extraction of the release pin which allows activating the auto injector. The activation surface of the auto injector remains accessible when the release pin is removed, and the front end of the injector is forced against the injection site.

Patent application US20170361021A1 describes an injector mechanism of one or more hazardous agents which presents a safety member located at the end opposite the needle exit, being removable by a set of aligned guides with the respective corresponding cavities in the outer housing to form a pressure fit between the safety system and the outer housing. The safety system prevents or reduces the probability of involuntary release of the injector device during, for example, the transport or handling of the injector. The safety system can be removed by the user to allow use without restrictions to the injector.

Patent EP2753382B1 describes a device for automatic injection of a single dose of a medication, equipped with a release device with deformable part for the actuation device. The syringe plunger is axially connected to a shaft comprising four flexible axial arms having an engaged toothed end in the perimeter of an opening formed on a lid contained at the housing end of the syringe. The sliding of the lid causes the arm ends to deform and the couplings to loosen from the perimeter of the opening. In this manner, the actuation device is activated. The auto injector further includes a safety device to avoid the accidental deforming of the arm ends and the activation of the actuation device, consisting of a pin which extends from the lid and penetrates the arms of the shaft to avoid them deforming.

Patent US8734393B2 describes an auto injector device for the administration of liquid medications which emphasizes that the actuation means which are easy to operate are liable to accidental administration of a medication which can be hazardous. To overcome this problem, some auto injector devices incorporate safety locks which avoid accidental activation. In these devices, the safety lock must be disengaged before the device can be activated using the common actuation means. The disengagement of a safety lock or similar mechanism adds an extra step to the actuation process. The extra step can be complicated and unintuitive and may not allow a sufficiently quick administration of the dose of the medication in an emergency wherein the patient can panic and/or suffer. To administer a dose of medication, the user must disengage the safety pins from the slots. To facilitate this process, the device is provided with a trigger comprising a button with a finger contact surface and axially extending slots. These slots extend up to the rear part of the device and are axially aligned with the safety pins (in their relaxed state) of the internal body. The finger contact surface extends through a slot on the rear part of the outer body allowing access by the user. The button is axially deviated backwards from the spring effect of the body, although other means can be used.

The injection in different parts of the body also appears to be a recurrent error in these devices of the state of the art, there existing the risk that the drug is not injected in the muscle and the needle reaches the bone, which can cause more complications and even infections. If the adrenaline is not administered intramuscularly, the absorption is much slower, as documented.

### Summary of the Invention

The present invention is related, in a first aspect, to a portable adrenaline auto injector device, comprising a button (2) positioned at opposite end to the exit side of a needle (11); a semi-rigid membrane (10) for protecting the button (2); a trigger (1) configured to be activated by the user who presses it against a surface; a support sheath (4) which houses an internal compression spring (5); a plunger (3) fitted in the support sheath (4); a lower compression spring (9) placed between the trigger (1) and the support (6); a safety system of the support (6) positioned between the support sheath (4) and the trigger (1); a cartridge (7) which contains adrenaline located within the support (6); an upper compression spring (14) positioned between the button (2) and the support sheath (4); two anti-return tabs (13) with rotating axis on the support (6); a needle (11) applied on the end of the cartridge (7); a needle (11) rubber protection (8); a casing (12) which houses all the internal components of the auto injector device; wherein the internal compression spring (5) is configured to push the plunger (3) and the cartridge (7).

The present invention is related, in a second aspect, to an operating method of the auto injector device as defined in the first aspect, characterized by comprising the following steps:
a. when pressed, the trigger (1) vertically pushes the internal mechanism of the auto injector device, including the plunger (3) and the cartridge (7) fitted in the support sheath (4); and
b. when pressed through the semi-rigid membrane (10), the button (2) displaces the plunger (3), causing it to disengage from the support sheath (4) and
c. once disengaged, the plunger (3) triggers the decompression of the internal compression spring (5), which pushes the safety system of the support (6), the cartridge (7) and the plunger (3), causing the needle (11) to perforate the rubber protection (8).

### Solution of the Technical Problems

The present invention is related to an adrenaline auto injector device with a tapered body having the reduction of the section towards the end of the needle to ensure the automatic reading of the side of the device which must be against the thigh muscle of the leg. To ensure that the adrenaline dose is only injected when the user is handling the equipment correctly, the outer shape obliges the user to interact with two parts sequentially which are located at opposite ends of the outer body. At the thinner end of the device there is found the needle protection capsule which unblocks the safety system, and on the opposite end (wider side) there is found the pressure button which triggers the needle. The pressure button is protected by a semi-rigid membrane which avoids accidental triggering and simultaneously allows access when it is intended to use the auto injector. This semi-rigid membrane constitutes the common system for protection and access to the button which triggers the administration of the adrenaline dose.

As a safety complement to the semi-rigid membrane, a spring is used to create tension between the button and the trigger, ensuring that the parts do not move accidentally and activate the trigger system inadvertently. This mechanism allows the device to be transported without the need for an additional protection container.

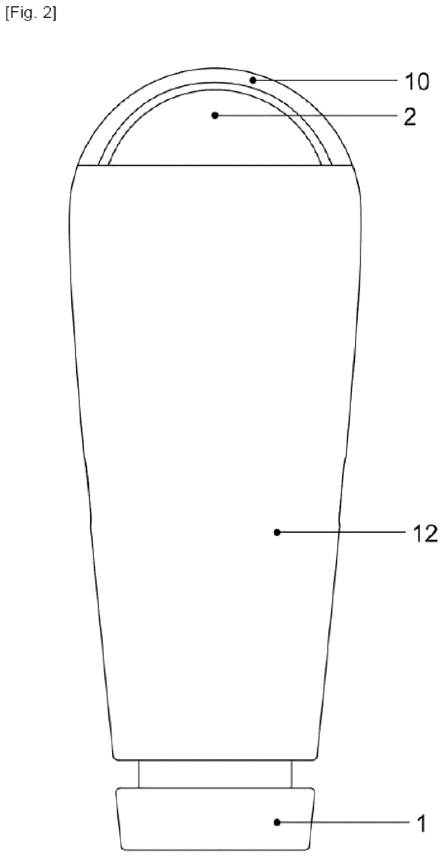

To reinforce the safety mechanisms of the device the outer body of the casing presents a tapered body which can be verified from Fig. 2, which presents its reduced section towards the needle end to promote the automatic reading of the side of the device which must be against thigh muscle of the leg (thinner side of the casing). To ensure that the adrenaline dose is only injected when the user is handling the equipment correctly, the outer shape obliges the user to interact with two parts sequentially which are located at the two opposite ends of the outer body. At the end of the needle side there is found the trigger (1) which when pressed against the thigh displaces axially to internally unblock the safety system of the support (6) and, subsequently, when the button (2) at the opposite end is pressed through the semi-rigid membrane (10), activates the compression spring (5) which activates the auto injector. If the button (2) is pressed without the trigger (1) having previously deactivated the safety system of the support (6), the auto injector is not activated because the axial movement of the button (2) is internally blocked.

The suggestion of use promoted by the tapered outer body of the auto injector which intuits the user about the end of the needle (11) represented by the thinner end of the casing (12) promotes that this be correctly handled; however, other redundancy mechanisms were exploited for the use of the device, such as: the indication of the exact place of application (thigh) and the necessary steps for the correct administration, concretely pressing the side end of the needle against the thigh and subsequently pressing the semi-rigid membrane (10) at the opposite end until the axial movement of the button (2) activates the auto injector.

In order to ensure that the user administers the adrenaline dose on the thigh, and not in another part of the body, there is jointly inserted with the utilization steps an illustrative image of the representation of the area of application on the human body in the act of administration.

### Advantageous Effects of the Invention

The auto injector adrenaline device presents a tapered outer body which conditions incorrect use and which presents a reduction in the number of steps that are necessary for applying the dose with resource to a polyurethane thermoplastic semi-rigid membrane which simultaneously protects and allows access to the button which triggers the adrenaline dose.

The present invention allows reducing the number of steps that are necessary for the safe use of the device since it does not require detachable parts normally used to block the inadvertent trigger system. The correct use guarantee is promoted by the suggestion of use of the outer shape and by the redundancy in visual use instructions.

It is a purpose of the present invention to ensure intuitive capability to the device for applying the emergency medication in individuals who are untrained in the use of injectors, both on themselves as on third parties, relatively to the solutions existing in the state of the art.

The present invention is useful for all people who are diagnosed with severe allergies or for whom adrenaline auto injectors are prescribed for treatment of allergic reactions, universally considered such as the first-line treatment for reducing the symptoms of anaphylaxis and prevention of the mortality in emergency situations.

The present invention presents a dose administration procedure with only two steps, contrary to the devices mentioned which add supplementary steps due to the need for removal of movable parts which ensure the blocking and protection system of the auto injector activation trigger.

The final shape of the device was developed from the dimension of the metacarpus, to ergonomically adapt to the palm of the user. The vertical symmetry allows the user, whether right or left-handed, to correctly handle the device, without ever covering the surface wherein there are placed the use instructions of the device.

### Brief Description of the Figures

With the purpose of providing an understanding of the principles according to the embodiments of the present invention, reference will be made to the embodiments illustrated in the figures and to the terminology used to describe them. In any case, it should be understood that there is no intention of limiting the scope of the present invention to the contents of the figures. Any subsequent alterations or modifications of the inventive characteristics illustrated herein, as well as any additional applications of the principles and embodiments of the invention illustrated, which would normally occur to a person skilled in the art having the knowledge of this specification, are considered as being within the scope of the claimed invention.

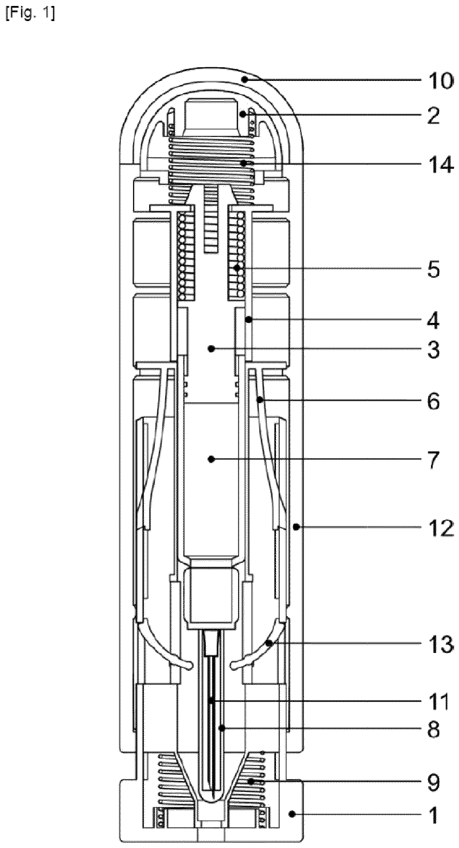

[Fig.1] Represents in schematic form the portable adrenaline auto injector device. This device is comprised by a trigger (1) which is located at the side edge of the needle (11), a drive button (2) of the mechanism which is found at the opposite end beside the needle, a plunger (3) which pushes the cartridge (7) with adrenaline dose, a support sheath (4) which houses the internal compression spring (5) which forces the penetration of the needle (11) in the thigh muscle of the leg, a safety system of the support (6) which maintains the system blocked before use, a rubber protection (8) of the needle (11), a lower compression spring (9) which axially displaces the trigger (1) to cover the needle (11) after use of the auto injector, a semi-rigid membrane (10) which protects the button (2) against inadvertent use, an outer casing (12) which ensures the movements described and the correct positioning of all the internal components of the auto injector, two anti-return tabs (13) which prevent the system from recovering the original position after the adrenaline dose is administered and an upper compression spring (14) which is constantly straining the button (2) towards the semi-rigid membrane (10) guaranteeing that the parts do not move accidentally and activate the trigger system inadvertently.
[Fig.2] Represents a front elevation of the adrenaline auto injector enhancing the tapered outer shape. This device is comprised by a trigger (1) which is found at the thinner end of the device, a drive button (2) of the mechanism which is found at the wider end of the mechanism, a semi-rigid membrane (10) which protects the button (2) from inadvertent use, an outer casing (12) which accommodates all the internal components of the auto injector.

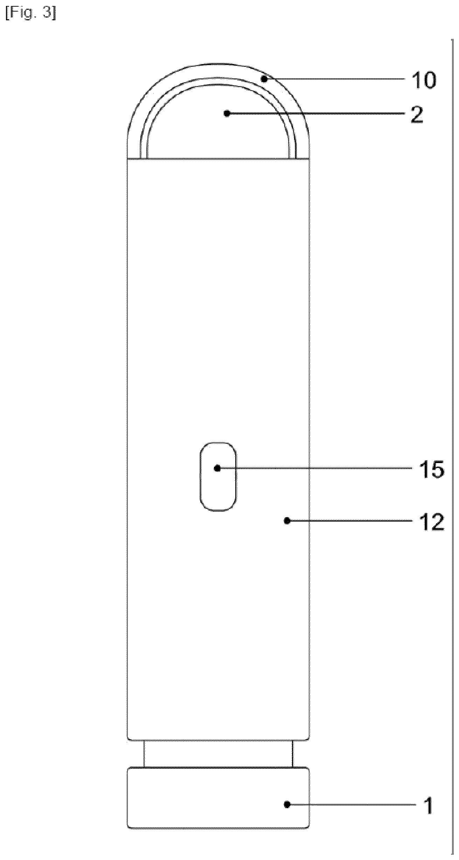

[Fig.3] Represents the adrenaline auto injector on the front elevation thereof enhancing the parallel outer shape and an orifice (15) for monitoring the state of the adrenaline solution. This device is comprised of a trigger (1) which is located at the end of the needle side, a drive button (2) of the mechanism which is located at the opposite end of the needle side, a semi-rigid membrane (10) which protects the button (2) from inadvertent use, an outer casing (12) which accommodates all the internal components of the auto injector.

### Description of the Embodiments of the Invention

The present invention is related to an adrenaline auto injector device with a tapered outer body which conditions incorrect use and which presents a reduction in the number of steps that are necessary for applying the dose with resource to a polyurethane thermoplastic semi-rigid membrane (10) which simultaneously protects and allows access to the button (2) which triggers the adrenaline dose.

The present invention presents a semi-rigid membrane (10) for protecting the button (2) which triggers the administration of the adrenaline dose. This semi-rigid membrane (10), positioned at the end opposite to the orifice for expelling the needle (11), has the double function of protecting the button (2) against inadvertent use and allowing, simultaneously, access to the button (2) at the moment of applying the adrenaline, without the need to interact with other movable parts of the device and ensuring the administration of the medication in only two safe steps.

As a protective complement to the semi-rigid membrane (10), there is resource to an upper compression spring (14) to create tension between the button (2) and the trigger (1), ensuring that the parts do not move accidentally and activate the trigger system inadvertently. The upper compression spring (14) positioned between the button (2) and the support sheath (4). This mechanism allows the device to be transported without the need for an additional protection container.

As illustrated in Fig. 1, the portable adrenaline auto injector device comprises the semi-rigid membrane (10) positioned over the button (2), and it is configured to protect said button (2). The button (2) is positioned at the opposite end beside the exit of a needle (11). The support sheath (4) houses an inner compression spring (5) and at least one portion of the plunger (3), which is fitted on the support sheath (4). The lower compression spring (9) is placed between the trigger (1) and the support (6). The safety system of the support (6) is positioned between the support sheath (4) and the trigger (1). The cartridge (7), which contains adrenaline, is located inside the support (6) and is connected to the plunger (3). The upper compression spring (14) is positioned between the button (2) and the support sheath (4). Preferably, the two anti-return tabs (13) with rotating axis on the support (6) are connected to the lower portion of the support (6). The needle (11) is connected to the cartridge end (7). The rubber protection (8) covers the needle (11). The casing (12) accommodates all the internal components of the auto injector device, wherein the internal compression spring (5) is configured to push the plunger (3) and the cartridge (7).

The working of the device comprises the following steps:
a) The trigger (1), when pressed against the leg, vertically pushes all the internal mechanism of the auto injector device, including the plunger (3) and the cartridge (7) fitted in the support sheath (4); and
b) The button (2), when pressed through the semi-rigid membrane (10), displaces the plunger (3), causing it to disengage from the support sheath (4); and
c) The disengaged plunger (3) triggers the decompression of the internal compression spring (5), which pushes the safety system of the support (6), the cartridge (7) and the plunger (3), causing the needle (11) to perforate the rubber protection (8) and the thigh muscle, being thereafter injected the adrenaline dose which is located inside the cartridge (7).

After the administration, there exists an automatic needle protection system which comes into action right after the needle (11) is injected. After administration of the adrenaline dose, the lower compression spring (9) placed between the trigger (1) and the safety system of the support (6) decompresses, pushing the trigger (1) until it covers the needle (11) completely. The trigger (1) is blocked in this position by the two anti-return tabs (13) which prevent it from returning to the original position.

In the preferred embodiments of the adrenaline auto injector device, the button (2) is configured to be pressed simultaneously with the trigger (1) to deactivate the safety system of the support (6).

In the preferred embodiments of the adrenaline auto injector device, the outer shape of the casing (12) is of a tapered body, which section is reduced towards the needle end.

In other preferred embodiments, the adrenaline auto injector device comprises a monitoring orifice (15) of the state of the adrenaline solution in the casing (12).

Preferably, the semi-rigid membrane (10) is made of thermoplastic polyurethane.

In other preferred embodiments, the adrenaline auto injector device comprises an automatic needle (11) protection system wherein the lower compression spring (9) placed between the trigger (1) and the safety system of the support (6) is configured to decompress and push the trigger (1) until it completely covers the needle (11).

Preferably, the trigger (1) is configured to be blocked in this position by the two anti-return tabs (13) which prevent it from returning to the original position.

As used throughout this patent application, the expression "or" is used in the inclusive sense instead of the exclusive sense, unless the exclusive sense is clearly defined in a specific situation. In this context, a sentence of the type "X uses A or B" must be interpreted as including all the pertinent inclusive combinations, for example "X uses A", "X uses B" and "X uses A and B".

As used throughout this patent application, the indefinite article "one" must be interpreted generally as "one or more", unless the sense of a singular embodiment is clearly defined in a specific situation.

The subject matter described above is provided as an illustration of the present invention and must not be interpreted so as to limit it. The terminology used with the purpose of describing specific embodiments, according to the present invention, must not be interpreted to limit the invention. As used in the specification, the definite and indefinite articles, in their singular form, aim at the interpretation of also including the plural forms, unless the context of the description indicates, explicitly, the contrary. It will be understood that the expressions "comprise" and "include", when used in this description, specify the presence of the characteristics, the elements, the components, the steps, and the related operations, however, they do not exclude the possibility of other characteristics, elements, components, steps, and operations also being contemplated.

All the alterations, providing that they do not modify the essential characteristics of the claims that follow, must be considered as being within the scope of protection of the present invention.

### List of Reference Indications

1. A trigger
2. A button
3. A plunger
4. A support sheath
5. An internal compression spring
6. A support
7. A cartridge
8. A rubber protection
9. A lower compression spring
10. A semi-rigid membrane
11. A needle
12. A casing
13. An anti-return tab
14. An upper compression spring
15. An orifice

### List of Citations

### Patent documents

North American patent US8870827B2 of Young Matthew Egerton et al., published on October 28, 2014;
North American patent application US20170361021A1 of Wotton Paul K. et al., published on December 21, 2017;
European Patent EP2753382B1 of Edhouse Mark Jeffrey et al., published on December 9, 2015;
North American patent US8734393B2 of Ian Cleathero, published on May 27, 2014.

## Claims

1. A portable adrenaline auto injector device, **characterized by** comprising a button (2) positioned at the opposite end to the side of exit of the needle (11); a semi-rigid membrane (10) for protecting the button (2); a trigger (1) configured to be activated by the user who presses it against a surface; a support sheath (4) which houses an internal compression spring (5); a plunger (3) fitted in the support sheath (4); a lower compression spring (9) placed between the trigger (1) and the support (6); a safety system of the support (6) positioned between the support sheath (4) and the trigger (1); a cartridge (7) which contains adrenaline located within the support (6); an upper compression spring (14) positioned between the button (2) and the support sheath (4); two anti-return tabs (13) with rotating axis on the support (6); a needle (11) applied on the end of the cartridge (7); a needle (11) rubber protection (8); a casing (12) which houses all the internal components of the auto injector device; wherein the internal compression spring (5) is configured to push the plunger (3) and the cartridge (7).

2. The device, according to the preceding claim, **characterized by** the button (2) being configured to be pressed simultaneously with the trigger (1) to deactivate the safety system of the support (6).

3. The device, according to any of the preceding claims, **characterized by** the outer shape of the casing (12) being a tapered body, which section is reduced towards the needle end.

4. The device, according to any of the preceding claims, **characterized by** comprising a monitoring orifice (15) of the state of the adrenaline solution in the casing (12).

5. The device, according to any of the preceding claims, **characterized by** the semi-rigid membrane (10) being made of thermoplastic polyurethane.

6. The device, according to any of the preceding claims, **characterized by** comprising an automatic needle (11) protection system wherein the lower compression spring (9) placed between the trigger (1) and the safety system of the support (6) is configured to decompress and push the trigger (1) until it completely covers the needle (11).

7. The device, according to the preceding claim, **characterized by** the trigger (1) being configured to be blocked by the two anti-return tabs (13) which prevent it from returning to the original position.

8. An operating method of the auto injector device, as defined in any of claims 1 to 7, **characterized by** comprising the following steps:
a. when pressed, the trigger (1) vertically pushes the internal mechanism of the auto injector device, including the plunger (3) and the cartridge (7) fitted in the support sheath (4); and
b. when pressed through the semi-rigid membrane (10), the button (2) displaces the plunger (3), causing it to disengage from the support sheath (4) and
c. once disengaged, the plunger (3) triggers the decompression of the internal compression spring (5), which pushes the safety system of the support (6), the cartridge (7) and the plunger (3), causing the needle (11) to perforate the rubber protection (8).
